# EUROPÄISCHE PATENTANMELDUNG

(11) **EP 3 750 504 A1**
(43) Veröffentlichungstag der Anmeldung: **16.12.2020**
(21) Anmeldenummer: 20180115.6
(22) Anmeldetag: 15.06.2020
(51) Int. Cl.: A61B 90/30, A61B 90/35

(54) **MOBILE CHIRURGISCHE LEUCHTE**

(30) Priorität: 14.06.2019 DE 102019116223
(71) Anmelder: Baumgartner, Heiko, 72074 Tübingen (DE)
(72) Erfinder: Baumgartner, Heiko, 72074 Tübingen (DE)
(74) Vertreter: Straub, Bernd

(57) **Zusammenfassung**

Die Erfindung betrifft eine chirurgische Leuchte 1. Sie ist mobil ausgebildet, so dass sie in das Operationsfeld eingebracht und wieder entnommen werden kann. Durch eine geeignete Positionierung ist es möglich, den relevanten Teil des Operationsfeldes bei Bedarf zu beleuchten und andererseits Beeinträchtigungen durch die chirurgische Leuchte 1 im Operationsfeld gering zu halten. Sie zeigt wenigstens eine Lichtquelle 2 zur Beleuchtung des Operationsfeldes und eine zugehörige elektrische Stromversorgung 3.

Die chirurgische Leuchte 1 ist mit einem plattenförmigen, runden Träger 5 mit wenigstens einer Lichtquelle 2 auf seiner Vorderseite 5a ausgebildet. Dadurch ist eine gute Lichtverteilung von der Vorderseite 5a in Richtung des Operationsfeldes gegeben und zudem durch den Träger 5 eine störende Blendung des Chirurgen bei der Betrachtung des Operationsfeldes mit der darin befindlichen chirurgischen Leuchte 1 weitgehend ausgeschlossen. Der runde Träger 5 ist mit einer Halterung 6,7 zur lösbaren Verbindung mit einem chirurgischen Instrument im Operationsfeld versehen und ermöglicht dadurch eine sichere und lösbare Verbindung der chirurgischen Leuchte über den steifen Träger 5 zu einem chirurgischen Instrument im Operationsfeld und eine bedarfsgemäße Positionierung der chirurgischen Leuchte 1 in Verbindung mit der guten Lichtverteilung bei reduziertem Blendrisiko. Die erfindungsgemäße chirurgische Leuchte 1 erweist sich zudem als besonders kompakt und robust.

## Beschreibung

### TECHNISCHES GEBIET

Die Erfindung betrifft eine mobile, chirurgische Leuchte.

### STAND DER TECHNIK

Aus der DE 19 923 402 A1 und aus der DE 10 2007 042 646 A1 sind stationäre OP-Leuchten bekannt, die ein Leuchtengehäuse zeigen, welches mit einer an der Decke des Operationssaales angebrachten Schwenkarmanordnung gehalten wird. In dem Leuchtengehäuse ist eine Reihe von LEDs zur Beleuchtung des Operationsfeldes angeordnet. Diese Operationsleuchten erweisen sich als sehr aufwendig und wenig flexibel. Sie neigen dazu, gerade bei Operationen in schlecht zugänglichen Körperöffnungen unerwünschten Schatten zu werfen und dadurch das Operationsfeld nicht perfekt auszuleuchten.

Der biegsame Lichtleiter des Deutschen Gebrauchsmusters DE 1 880 706 U ermöglicht das Trennen der Lichtquelle von dem Ort der Lichtabgabe am distalen Ende eines Endoskops. Dadurch gelingt es, dass der Chirurg durch die Beleuchtungseinrichtung praktisch nicht mehr behindert wird. Weiterhin gelingt es, durch die getrennte Anordnung der Lichtquelle starke Lichtquellen zu verwenden, damit ausreichend Licht in die Körperhöhle als Operationsfeld gebracht wird. Dennoch gehen auf dem Weg von der abgesetzten, starken Lichtquelle zum Operationsfeld relevante Lichtmengen verloren und der biegsame Lichtleiter als Transportmedium erweist sich als sehr anfällig.

Aus dem Deutschen Gebrauchsmuster DE 20 2017 102 966 U1 ist eine Stirnlampe zum Ausleuchten eines Arbeitsfeldes bekannt, die eine Leuchte, einen Akkumulator und eine Halterung für den Akkumulator aufweist, die dazu ausgebildet ist, den Akkumulator so am Kopf des Trägers zu positionieren, dass die Gewichtskraft des Akkumulators auf die Muskulatur unter der Protuberantia occipitalis externa übertagen wird. Diese Stirnlampe zeigt ebenso den Nachteil, dass das Operationsfeld durch die benutzten Instrumente zumindest partiell verschattet ist und dadurch der Chirurg behindert wird. Zudem ist es notwendig, dass der Chirurg stets die Kopfposition und -orientierung so wählt, dass das Operationsfeld ausreichend beleuchtet ist.

Weiterhin ist aus der WO 2005/117686 A1 ein Laryngoskop bekannt. Dieses chirurgische Instrument zeigt eine Lichtquelle am vorderen Ende des Laryngoskopspatels, so dass im Betrieb in den Mund- beziehungsweise Rachenraum vor dem Spatelende Licht abgestrahlt wird. Durch das Anordnen der Lichtquelle am Instrument ist eine gute Beleuchtung ausschließlich für dieses eine Instrument und damit ausschließlich für die zu diesem Instrument gehörige eine Operationssituation gegeben.

Der aus der EP 2 395 903 B1 bekannte beleuchtete zahnärztliche Retraktor gewährleistet das Offenhalten und Beleuchten der Mundhöhle eines Patienten. Er zeigt zwei Wangenhalter, die mehrere Leuchtdioden so aufweisen, dass sie in einer Reihe angeordnet sind und vom Wangenhalter in die Mundhöhle des Patienten leuchten können. Dieses Instrument erfüllt zwei Funktionen zugleich, nämlich das Offenhalten und das Beleuchten der Mundhöhle, so dass der Zahnarzt in dieser Mundhöhle seine zahnärztlichen, insbesondere chirurgischen Aufgaben erfüllen kann. Dieses spezielle Instrument ist sehr aufwendig aufgebaut und nur für den dentalen Einsatz geeignet.

Aus dem US-Patent US 9,259,288 B2 ist eine Anordnung zur Beleuchtung und Unterstützung von Operationen bekannt, bei der mehrere Elemente mit Magneten und LEDs zu beiden Seiten des Hautgewebes also zum einen im Körper und zum anderen außerhalb des Körpers mittels Magnetkraft gehalten werden. Diese Anordnung erweist sich als sehr aufwendig in der Handhabung.

Aus der US-Anmeldung US 2008/0278936 A1 ist eine chirurgische Leuchte bekannt, welche in einem geschlossenen Gehäuse eine Batterie, eine Leiterplatte mit Chips und eine LED als Lichtquelle zeigt. Mit Hilfe einer selbstklebenden Fläche am Gehäuse wird die Leuchte im Operationsfeld befestigt. Mittels eines Druckschalters am Gehäuse lässt sich die chirurgische Leuchte bedienen. Diese chirurgische Leuchte erweist sich als recht groß und unhandlich sowie in der Handhabung als umständlich.

Aus der US-Anmeldung US 2005/0099824 A1 ist ein Verfahren und ein System für eine chirurgische Leuchte bekannt, welche eine in einem Gehäuse angeordnete Batterie sowie eine oder mehrere Leiterplatten mit darauf angeordneten LEDs zeigt. Das Gehäuse ist mit einer Halterung versehen, mittels der die chirurgische Leuchte an einem anderen Gegenstand befestigt werden kann. Die Steuerung erfolgt über ein abgesetztes Bedienfeld über einen Steuerbus. Diese chirurgische Leuchte erweist sich als recht groß und unhandlich sowie in der Handhabung als umständlich.

### BESCHREIBUNG DER ERFINDUNG

Der Erfindung liegt die Aufgabe zu Grunde, eine gegenüber dem Stand der Technik verbesserte chirurgische Leuchte anzugeben.

Die Aufgabe wird erfindungsgemäß mit einer Vorrichtung gelöst, welche die im Anspruch 1 angegebenen Merkmale aufweist.

Vorteilhafte Ausgestaltungen der Erfindung sind Gegenstand der Unteransprüche.

Die erfindungsgemäße chirurgische Leuchte ist nicht stationär sondern mobil ausgebildet, so dass sie in das Operationsfeld eingebracht und aus diesem wieder entnommen werden kann. Sie weist wenigstens eine Lichtquelle zur Beleuchtung des Operationsfeldes auf und ist mit einer elektrischen Stromversorgung für die wenigstens eine Lichtquelle verbunden. Durch das Vorsehen einer lösbaren Befestigung ist es möglich, die chirurgische Leuchte an einem chirurgischen Instrument oder einem Gegenstand im Operationsfeld zu befestigen und von dort auch wieder zu lösen und zu entnehmen. Dadurch ist es möglich, den relevanten Teil des Operationsfeldes bei Bedarf zu beleuchten und andererseits Beeinträchtigungen durch die chirurgische Leuchte im Operationsfeld besonders gering zu halten.

Die chirurgische Leuchte ist mit einem Träger zur Aufnahme der wenigstens einen Lichtquelle versehen, wobei der Träger im Wesentlichen als Leiterplatte mit einer Vorderseite und einer zweiten Seite ausgebildet ist. Vorteilhafterweise ist auf der Vorderseite der Leiterplatte die wenigstens eine Lichtquelle angeordnet, so dass eine kompakte, leichte und gute Lichtverteilung von der Vorderseite in Richtung des Operationsfeldes gegeben ist und zudem durch die Schutzwirkung der Leiterplatte beziehungsweise des Trägers eine störende Blendung des Chirurgen bei der Betrachtung des Operationsfeldes mit der darin befindlichen chirurgischen Leuchte weitgehend ausgeschlossen ist. Dadurch, dass der Träger mit einer Halterung zur lösbaren Verbindung mit einem chirurgischen Instrument oder einem Gegenstand im Operationsfeld versehen ist, ist es möglich, eine sichere und lösbare Verbindung der chirurgischen Leuchte über den steifen Träger beziehungsweise die steife Leiterplatte zu einem chirurgischen Instrument oder einem Gegenstand im Operationsfeld zu schaffen und dadurch die bedarfsgemäße, vorteilhafte Positionierung der chirurgischen Leuchte in Verbindung mit der guten Lichtverteilung mit sehr reduziertem Blendrisiko zu ermöglichen. Die erfindungsgemäße chirurgische Leuchte erweist sich zudem als besonders robust.

Darüber hinaus hat es sich besonders bewährt, die chirurgische Leuchte so auszugestalten, dass der Träger als Leiterplatte mit im Wesentlichen rundem insbesondere ovalem oder kreisrundem Querschnitt ausgebildet ist. Durch diese flächige Ausbildung der Leiterplatte gelingt es, die Handhabung der chirurgischen Leuchte im Operationsfeld besonders sicher zu gestalten, da eine Beeinträchtigung der Aktivitäten des oder der Chirurgen im Operationsfeld durch die chirurgische Leuchte reduziert ist. Vorzugsweise liegt die flächige Ausdehnung der Leiterplatte im Bereich von wenigen Zentimetern, so dass es einerseits gelingt, das Gewicht der chirurgischen Leuchte gering zu halten und andererseits Beeinträchtigungen des Sichtfeldes durch die chirurgische Leuchte gering zu halten und dadurch die Handhabung der Leuchte besonders vorteilhaft zu gestalten. Dies gilt umso mehr, da durch diese Ausbildung der chirurgischen Leuchte auf ein Gehäuse, das den runden Träger beziehungsweise die runde Leiterplatte schützend umschließt, verzichtet werden kann und dadurch ein besonders kompakter und robuster Aufbau ermöglicht ist.

Weiterhin hat es sich bewährt, die chirurgische Leuchte mit der Halterung so weiterzubilden, dass sie wenigstens ein Loch im Träger aufweist, wobei das wenigstens eine Loch als Teil der Halterung für die chirurgische Leute wirkt und geeignet ist, eine Befestigung an einem chirurgischen Instrument zu ermöglichen, wobei das chirurgische Instrument vorzugsweise in das Loch eingebracht und von dem Träger umschlossen und dadurch geführt wird, was eine sichere und einfache Halterung ermöglicht.

Gerade das Vorsehen wenigstens eines Loches im Träger beziehungsweise in der Leiterplatte ermöglicht es, ein schaftförmiges Instrument insbesondere ein Sauginstrument durch das Loch zu führen und dadurch die chirurgische Leuchte an dem betreffenden Instrument zu befestigen beziehungsweise entlang des Schaftes zu verschieben. Dies ist insbesondere dann besonders vorteilhaft ermöglicht, wenn der Durchmesser des Loches etwa dem Außendurchmesser des Schaftes entspricht insbesondere wenig geringer ist. Zudem hat es sich besonders bewährt, das Loch der Leuchte mit einer elastischen Innenverkleidung zu versehen, die ein federndes Klemmen und Lösen der Leuchte zur Verschiebung und Neupositionierung beziehungsweise zur Entfernung besonders einfach handhabbar und sicher ermöglicht. Vorzugsweise wird die elastische Innenverkleidung dabei ringförmig das Loch umschließend ausgebildet.

Ergänzend oder alternativ hat es sich besonders bewährt, wenigstens einen Clip, wenigstens einen Magneten, wenigstens ein Formschlusselement, wenigstens ein elastisches Halteelement, wenigstens eine Klebefläche und/oder wenigstens ein Klettverschlusselement als Teil der Halterung vorzusehen.

Dabei hat es sich bewährt, insbesondere ergänzend zu dem Loch mittels wenigstens eines Clips, der rastend das Instrument oder den Gegenstand im Operationsfeld aufnimmt und dadurch eine sichere und lösbare sowie einfach zu handhabende Halterung für die chirurgische Leuchte schafft, eine einfache und gute Handhabung zu ermöglichen.

Daneben hat es sich insbesondere ergänzend zu dem Loch bei Verwendung eines oder mehrerer Magnete als Teil der Halterung als vorteilhaft erwiesen, dass die anziehende Wirkung auch mit geringem Abstand von dem Instrument oder Objekt, an das die Leuchte befestigt werden soll, und damit nicht erst bei engem Kontakt zur Wirkung kommt. Hierdurch ist eine Führungsfunktion oder Leitfunktion bereits bei einem gewissen Abstand ermöglicht.

Auch das Vorsehen von an das zu verbindende Instrument oder Objekt im Operationsfeld angepassten Ausnehmungen insbesondere in Form eines Loches zur formschlüssigen Aufnahme, die vorzugsweise im Randbereich angeordnet sind, hat sich als vorteilhaft erwiesen, denn diese ermöglichen eine einfache Handhabung, insbesondere wenn diese formschlüssigen Ausnehmungen zusätzlich mit einem elastischen insbesondere gummielastischen Material versehen sind und dadurch eine zusätzliche klemmende Wirkung erreicht werden kann.

Alternativ oder ergänzend haben sich insbesondere ergänzend zu dem Loch auch Klebeflächen oder Klettverschlüsse als Teil der Halterung bewährt, da diese regelmäßig keine exakte Positionierung der Leuchte gegenüber dem Instrument beziehungsweise dem Objekt erfordern und dadurch eine unkomplizierte Handhabung ermöglichen.

Daneben haben sich auch Kombinationen dieser verschiedenen Elemente zur Ausbildung einer Halterung bewährt, wobei sich gerade Kombinationen eines Loches mit einer oder mehreren anderen alternativen oder kumulativen Elementen einer Halterung bewährt haben. Beispielsweise hat es sich als besonders vorteilhaft herausgestellt, dass im Randbereich eine sich konisch verjüngende Ausnehmung insbesondere in Form eines Loches zur Aufnahme des Instrumentes beziehungsweise des Objektes, an welches die Leuchte im Operationsfeld befestigt werden soll, vorgesehen ist. Diese Ausnehmung ist bevorzugt mit einem den Träger und damit auch die Ausnehmung umschließenden gummielastischen Ring oder einem Halteband versehen, das mit einer Klettverschlussverbindung zusammenwirkt und geeignet ist, die Ausnehmung mit dem aufgenommenen Instrument positionsfixierend zu verschließen. Die Positionierung des Instruments oder des Objekts in der Ausnehmung wird zusätzlich durch eine fakultative gummielastische Auskleidung der Ausnehmung verbessert.

Dabei hat es sich besonders bewährt, die chirurgische Leuchte so weiterzubilden, dass die Leiterplatte mit einer Dicke von wenigen Millimetern gebildet ist. Durch diese bevorzugte Dicke, die auch als Stärke der Leiterplatte bezeichnet werden kann, gelingt es, einerseits eine ausreichend steife und andererseits eine leichte und kompakte chirurgischen Leuchte zu schaffen, die sich durch eine besonders gute Handhabung bei guter Lichtverteilung auszeichnet. Dies ist in besonderem Maße dann gegeben, wenn die Leiterplatte aus Metall, aus Kunststoff wie zum Beispiel Teflon und/oder Keramik wie zum Beispiel aus einer Aluminiumdioxid-Keramik gebildet ist. Vorzugsweise werden dabei nicht-poröse Werkstoffe verwendet, die einerseits als Leiterplattengrundlage, also als Träger zum Aufbringen von elektrischen Leitungen und Anordnen von elektronischen Komponenten dienen, und andererseits geeignet sind, ausreichend sterilisiert zu werden beispielsweise im Rahmen eines Autoklavierprozesses ohne wesentliche Materialbeeinträchtigung. Weiterhin hat es sich bewährt die Leiterplatte mehrteilig auszubilden und über flexible oder semiflexible Abschnitte miteinander zu verbinden. Dadurch wird es möglich die flächige insbesondere laterale Ausdehnung der leiterplatte zu reduzieren und dadurch die Abschattung des Operationsbereiches durch die chirurgische Leuchte zu reduzieren. Dies lässt sich auch durch die Verwendung von flexiblen oder semiflexiblen Leiterplatten oder Teilen erreichen.

Als besonders bevorzugte erfindungsgemäße Vorrichtung hat sich eine chirurgische Leuchte gezeigt, die auf der zweiten Seite insbesondere auf der Rückseite des Trägers die Stromversorgung und/oder eine Steuerung der Leuchte aufweist. Durch diese Verteilung der genannten Komponenten der chirurgischen Leuchte gelingt es, den Querschnitt beziehungsweise die Ausdehnung der Leiterplatte beziehungsweise des Trägers gering zu halten und dadurch die Handhabung der Erfindung zu verbessern. Auch wird es möglich, auf der Vorderseite die Anzahl der Lichtquellen zur Beleuchtung des Operationsfeldes ohne wesentliche Erhöhung des Risikos einer Blendung zu erhöhen. Durch die vorteilhafte Anordnung auf der zweiten Seite beziehungsweise auf der Rückseite gelingt es zudem, die Stromversorgung beispielsweise über eine vorgesehene Kontaktstelle beispielsweise über einen Stecker, in den ein elektrisches Kabel zur Stromversorgung eingebracht werden kann, besonders vorteilhaft und wenig störend anzuordnen und dadurch die Handhabung der chirurgischen Leuchte besonders sicher zu gestalten. Durch die Möglichkeit, eine Mehrzahl von Lichtquellen auf der Vorderseite zur Beleuchtung des Operationsfeldes zu verwenden, wird es in besonderem Maße möglich, das Operationsfeld sehr effizient und gleichmäßig auszuleuchten.

Dabei ist die Steuerung der Leuchte oder ein Teil der Steuerung auf der Rückseite vorzugsweise so ausgebildet, dass sie die wenigstens eine Lichtquelle insbesondere alle Lichtquellen auf der Vorderseite des Trägers beziehungsweise der Leiterplatte ein- beziehungsweise ausschalten, in ihrer Helligkeit regeln und/oder in ihrer Frequenzverteilung - auch Wellenlängenverteilung genannt - variieren kann. Als besonders vorteilhaft hat es sich dabei erwiesen, eine oder mehrere selektiv ansteuerbare Lichtquellen so anzusteuern, dass bestimmte Frequenzen des Lichts selektiv abgegeben werden, die zum Beispiel im Rahmen der Tumorerkennung oder -markierung durch spezifische Farbstoffe besonders relevant sind. Dadurch können Tumore beziehungsweise Tumorareale verlässlich von nichtbefallenen Arealen des Körpers im Operationsfeld unterschieden werden.

Daneben hat es sich auch bewährt, den Träger der chirurgischen Leuchte mit wenigstens einer chemisch inerten und/oder flüssigkeitsdichten und/oder sterilisierbaren Kapselung für die wenigstens eine Lichtquelle, die Stromversorgung, die Steuerung und/oder Teile davon zu versehen. Die Kapselung erfolgt dabei bevorzugt mit Hilfe einer Vergussmasse, die die gekapselten Teile der chirurgischen Leuchte direkt ohne wesentliche Zwischenräume umschließt und kapselt. Die Kapselung umschließt damit den Träger der chirurgischen Leuchte nicht in der Art eines eigenständigen insbesondere beabstandeten Gehäuses. Dadurch gelingt es, einen sicheren Operationsbetrieb gerade bei einer mehrfachen Verwendung der chirurgischen Leuchte in verschiedenen Operationen mit zwischengeschalteter Sterilisierung beispielsweise durch Autoklavierung sicherzustellen. Aber auch bei einer Einmalverwendung und anschließender Vernichtung der chirurgischen Leuchte gelingt es, das Risiko einer Beeinträchtigung des Patienten zu verringern und dadurch eine besonders sichere Handhabung der chirurgischen Leuchte zu ermöglichen. Dabei hat sich gerade die Kombination aus einer chemisch inerten und flüssigkeitsdichten und sterilisierbaren Kapselung besonders bewährt. Vorzugsweise wird die Kapselung dabei insbesondere durch eine Vergussmasse aus Kunststoff vorzugsweise aus transparentem Polyurethan (PU) beziehungsweise aus einem coextrudierten Polycarbonat gebildet, welches nicht nur aufgrund seiner mechanischen und chemischen wie biologischen Kapselung besonders geeignet ist sondern auch aufgrund seiner optischen Eigenschaften die Möglichkeit zur Gestaltung der Lichtverteilung der durch die gekapselte Lichtquelle ausgestrahlten Strahlung schafft.

Daneben hat es sich bewährt, auf der zweiten Seite insbesondere auf der Rückseite des Trägers der chirurgischen Leuchte wenigstens eine Zusatzlichtquelle anzuordnen, wobei diese Zusatzlichtquelle insbesondere eine deutlich geringere Lichtstärke als die Lichtquelle aufweist. Bevorzugt wird diese wenigstens eine Zusatzlichtquelle durch die vorgesehene Steuereinheit so gesteuert, dass diese nur bei Bedarf eingeschaltet und dabei in ihrer Helligkeit nur bedarfsgemäß geregelt wird. Dies führt dazu, dass die wenigstens eine Zusatzleuchte regelmäßig nicht aktiv ist beziehungsweise so schwach leuchtet, dass eine unerwünschte Blendung des Chirurgen, der in das Operationsfeld mit der chirurgischen Leuchte blickt, gering ist.

Auch hat es sich bewährt, die chirurgische Leuchte mit einer Stromversorgung unter Verwendung eines elektrischen Kabels und/oder einer drahtlosen, induktiv gekoppelten Verbindung und/oder eines elektrischen Energiespeichers insbesondere eines Akkumulators oder eines Kondensators (z.B. Supercap oder Goldcap) weiterzubilden.

Gerade durch ein Vorsehen einer drahtlosen, induktiv gekoppelten Verbindung zur Übertragung von elektrischer Energie zur Betreibung der wenigstens einen Lichtquelle beziehungsweise zur Übertragung von Steuersignalen ist es möglich, auf das Vorsehen von Kabeln zur chirurgischen Leuchte zu verzichten und dadurch die Benutzung in dem schwierigen Umfeld des Operationsfeldes einfach und sicherer zu gestalten. Zudem wird das Risiko eines Verhedderns mit dem Kabel reduziert wie auch eine unerwünschte Schattenbildung im Operationsfeld durch das oder die Kabel verhindert. Dies wird zusätzlich durch das Vorsehen eines fakultativen elektrischen Speichers verbessert, der in der Lage ist, genügend viel elektrische Energie zu puffern, um relevante Zeitabschnitte einer Operation auch ohne Aufladung zu überbrücken. Dieser Vorteil tritt sowohl bei einer drahtlosen als auch bei einer kabelgebundenen elektrischen Energieversorgung ein. Durch das Vorsehen von elektrischen Kabeln zur Energieversorgung beziehungsweise zur Ansteuerung der chirurgischen Leuchte ist es möglich, die chirurgische Leuchte über einen längeren Zeitraum zu betreiben, ohne dass es zu einer relevanten Belastung durch elektromagnetische Strahlung beispielsweise durch die induktive Kopplung kommt.

Nach einer bevorzugten Weiterbildung der Erfindung wird die wenigstens eine Lichtquelle zur Beleuchtung des Operationsfeldes der chirurgischen Leuchte als wenigstens eine Laserdiode, wenigstens eine OLED und/oder wenigstens eine LED insbesondere als wenigstens ein ungehäuster LED-Chip ausgebildet. Durch das Vorsehen dieser besonderen Lichtquellen gelingt es, eine energieeffiziente, chirurgische Leuchte zu realisieren, die sich durch eine geringe Wärmeentwicklung im Bereich des Operationsfeldes auszeichnet und dabei eine gute Lichtverteilung in dem gewünschten Bereich des Operationsfeldes ermöglicht, ohne dass auf dem Weg zum Operationsfeld wesentliche Anteile der Lichtenergie verloren gehen. Dadurch gelingt es, diese chirurgische Leuchte besonders kompakt auszubilden und dadurch einen Einsatz unmittelbar im Operationsfeld zu ermöglichen. Gerade durch das Vorsehen von ungehäusten LEDs auf der Vorderseite des Trägers beziehungsweise der Leiterplatte, gelingt es, eine besonders kompakte chirurgische Leuchte bei vorteilhafter Lichtstärke und Lichtverteilung zu realisieren. Durch das Vorsehen von Laserdioden gelingt es, eine sehr farbselektive Lichtausstrahlung zu erreichen, was beispielsweise bei der Markierung und Darstellung von Tumorzellen im Gewebe von besonderer Bedeutung sein kann. In diesem Falle sind die Tumorzellen durch die Behandlung von fluoreszierenden Farbstoffen aktiviert worden und werden durch die selektive Farbverteilung beziehungsweise Frequenzverteilung oder Wellenlängenverteilung des Lichts der chirurgischen Leuchte aufgrund des Fluoreszenzverhaltens der Tumorzellen angesprochen, während normales Gewebe nicht angesprochen wird. Dies führt zu einer erfolgreichen und sicheren Unterscheidung tumorbehafteter Zellen von anderen, gesunden Zellen.

Dabei hat es sich besonders bewährt, bei mehreren Laserdioden, OLEDs und/oder LEDs insbesondere mehreren ungehäusten LED-Chips auf der Oberfläche insbesondere auf der Vorderseite des Trägers diese voneinander beabstandet und verteilt anzuordnen. Durch den dadurch entstandenen Array aus Lichtquellen lässt sich eine besonders gute Lichtverteilung und eine hohe Lichtstärke bei ausgeprägter Energieeffizienz und geringer Wärmeentwicklung realisieren. Dies wird im besonderen Maße dadurch ergänzt, wenn ungehäuste LEDs oder insbesondere OLEDs oder Laserdioden mit ihren geringen Abmessungen verwendet werden. Dadurch gelingt es zudem, besonders kompakte chirurgische Leuchten zu schaffen.

Vorzugsweise werden diese bevorzugten, mehreren Lichtquellen mit einer gemeinsamen Kapselung versehen, die insbesondere chemisch inert und/oder flüssigkeitsdicht und/oder sterilisierbar ist. Durch diese gemeinsame Kapselung ist eine vorzugsweise sterilisierbare beziehungsweise autoklavierbare Realisierung der chirurgischen Leuchte mit den gemeinsam gekapselten Lichtquellen geschaffen, was das Risiko einer Infektion des Patienten reduziert. Diese gemeinsame Kapselung ermöglicht eine besonders sichere Handhabung der chirurgischen Leuchte, die sich zudem aufgrund der kompakten Ausbildung als besonders gut handhabbar erweist.

Auch hat es sich als besonders vorteilhaft erwiesen, die Kapselung so auszubilden, dass das von der wenigstens einen Lichtquelle ausgesandte Licht durch die optischen Eigenschaften der Kapselung spezifisch umgelenkt wird und dadurch die Kapselung als optisches Element zur Lenkung insbesondere zur Bündelung des Lichts der wenigstens einen Lichtquelle wirkt. Dadurch ist es möglich, die gewünschte Lichtverteilung der wenigstens einen Lichtquelle auf der Vorderseite spezifisch insbesondere gleichmäßig zu gestalten und dadurch eine sehr effiziente, angenehme und sichere Beleuchtung des Operationsfeldes durch die chirurgische Leuchte zu gewährleisten. Dies wird durch die Verwendung von optisch transparentem Material für die Kapselung mit spezifischem Brechungsindex und spezifisch angepasster Form ermöglicht.

Besonders nützlich hat es sich dabei erwiesen, eine Bedieneinheit für die chirurgische Leuchte vorzusehen, die auf dem Träger und/oder in einer elektrischen Leitung zur Versorgung der chirurgischen Leuchte mit elektrischer Energie und/oder bei einer abgesetzten elektrischen Stromversorgung für die restliche chirurgische Leuchte angeordnet ist. Durch das Vorsehen der Bedieneinheit auf oder an dem Träger beziehungsweise der Leiterplatte gelingt es, eine kompakte, geschlossene chirurgische Leuchte zu realisieren. Wird die Bedieneinheit im Bereich der elektrischen Energieerzeugung und damit bei einer abgesetzten elektrischen Stromversorgung für die restliche Leuchte realisiert, die entweder kabelgebunden über ein elektrisches Kabel oder drahtlos über eine induktive Kopplung elektrische Energie von der Energieerzeugung an die abgesetzte chirurgische Leuchte mit der wenigstens einen Lichtquelle mit Steuereinheit überträgt, gelingt es, eine besonders einfach zu handhabende und zu steuernde chirurgische Lichtquelle zu ermöglichen. Darüber hinaus hat es sich alternativ auch bewährt, die Bedieneinheit zwischen der Energieerzeugung und dem Träger der chirurgischen Leuchte also auf dem Übertragungsweg der elektrischen Energie anzuordnen.

Eine bevorzugte Weiterbildung der erfindungsgemäßen chirurgische Leuchte zeigt eine Bedieneinheit für die Leuchte mit wenigstens einem Bedienschalter, der insbesondere einen mechanischen Bedienschalter, einen Berührschalter und/oder einen Annäherungsschalter aufweist, und/oder die als abgesetzte Bedieneinheit kabellos - zum Beispiel mittels Bluetooth oder WLAN - zum Ansteuern der Steuerung der chirurgischen Leuchte mit dieser verbunden ist. Diese ermöglichen eine gute Handhabung der chirurgischen Leuchte, ohne dass in das Operationsfeld insbesondere manuell eingegriffen und dieses Operationsfeld potentiell beeinträchtigt oder kontaminiert werden muss.

Auch hat es sich bewährt die Steuerung in Teile aufzuteilen und ein Teil auf dem Träger und damit unmittelbar in der Nähe der wenigstens einen Lichtquelle anzuordnen und dadurch eine gute und sichere Ansteuerung der wenigstens einen Lichtquelle zu ermöglichen, während der oder die anderen Teile der Steuerung abgesetzt vom Träger inbesondere außerhalb des Operationsfeldes angeordnet sind und eine Bedienung der chirurgischen Leuchte auf einfache, verlässliche und sichere Weise ermöglichen. Durch das Vorsehen eines Annäherungsschalters an der Bedieneinheit oder einem abgesetzten Teil der Steuerung ist eine besonders einfache Bedienung der chirurgischen Leuchte auch im Operationsfeld ermöglicht. Durch das Vorsehen ist im Hinblick auf eine sichere Bedienung eine bevorzugte chirurgische Leuchte geschaffen.

Die Erfindung wird nachfolgend anhand bevorzugter Ausführungsbeispiele unter Bezugnahme auf die Abbildungen beispielhaft erläutert. Die Erfindung ist nicht auf diese bevorzugten Ausführungsbeispiele beschränkt.
- Fig. 1: zeigt in einer schematischen Darstellung eine beispielhafte chirurgische Leuchte in einer Ansicht auf die dem Operationsfeld zugewandten Vorderseite,
- Fig. 2: zeigt eine schematische Ansicht der chirurgischen Leuchte aus Fig. 1 in einer Schnittdarstellung und
- Fig. 3: zeigt eine schematische Darstellung der chirurgischen Leuchte aus Fig. 1 in einer Ansicht auf die dem Chirurgen zugewandten Rückseite.

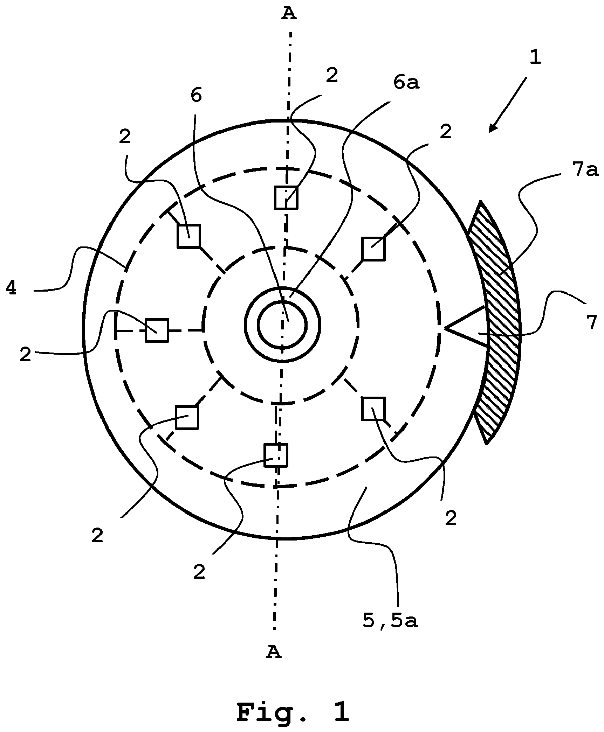

In Fig. 1 ist schematisch eine beispielhafte chirurgische Leuchte 1 dargestellt. Diese zeigt eine im Wesentlichen kreisrunde Leiterplatte 5, die als Träger 5 für verschiedene Komponenten der chirurgische Leuchte 1 dient und die eine Vorderseite 5a und eine Rückseite 5b aufweist. Die Leiterplatte 5 ist dabei aus Teflon beziehungsweise alternativ aus Aluminiumdioxid-Keramik gebildet, so dass diese scheibenförmige Leiterplatte 5 mit einer Dicke, auch Stärke genannt, von wenigen Millimetern und einem Durchmesser von etwa 3 Zentimetern besonders kompakt und leicht zu handhaben insbesondere mobil ist. Sie zeigt kein die runde, scheibenförmige Leiterplatte 5 umschließendes Gehäuse. Durch die verwendeten Komponenten und deren Anordnung beziehungsweise Ausbildung erweist sich diese chirurgische Leuchte 1 als besonders robust und wenig anfällig, was die Operationssicherheit besonders erhöht.

In Fig. 1 ist in einer Draufsicht die Vorderseite 5a der Leiterplatte 5 dargestellt, das ist die Seite der scheibenförmigen Leiterplatte 5, die dem zu beleuchtenden Operationsfeld zugewandt beziehungsweise welche dem Chirurgen abgewandt ist. Die Vorderseite 5a zeigt dabei gleichmäßig über die Fläche verteilt sieben einzelne Lichtquellen 2, die über elektrische Leitungen, auch Leiterbahnen 4 genannt, mit Strom versorgt werden und als ungehäuste LEDs 2 realisiert sind. Diese ungehäusten LEDs 2 werden mit der Leiterplatte 5a und den Leiterbahnen 4 fest verbunden, was insbesondere durch Bond-Prozesse erfolgt. Die einzelnen ungehäusten LEDs 2 werden zusätzlich mittels einer Kapselung mittels einer Vergussmasse vor äußeren Einflüssen insbesondere im Rahmen von Sterilisierungsprozessen wie Autoklavierprozessen oder im Rahmen der Operation geschützt. Die Kapselung bewirkt dabei auch umgekehrt, dass eine Kontaminierung des Operationsfeldes durch die durch die Kapselung geschützten Oberflächen erfolgen kann. Durch die Wahl des Kapselungsmaterials als optisch transparentes Material und die geeignete Wahl der Gestaltung der Kapselung gelingt es, Einfluss auf die Lichtverteilung des Lichtes der einzelnen gekapselten ungehäusten LEDs 2 zu nehmen und dadurch ein sehr gleichmäßiges, großflächiges und wenig verschattetes Leuchtfeld für die chirurgische Leuchte 1 zu erzeugen und dadurch ein sehr angenehme, leuchtstarke, ausgeglichene Beleuchtung des Operationsfeldes für den Chirurgen zu schaffen, ohne dass der Chirurg durch die Lichtquellen 2 auf der Vorderseite 5a der chirurgischen Leuchte 1 geblendet werden kann, da der Träger 5 beziehungsweise die Leiterplatte 5 das von der chirurgischen Leuchte 1 erzeugte Licht in Richtung des Chirurgen abblockt und dadurch ein störendes Blenden verhindert. Ein solches Blenden würde ein unerwünschtes Ermüden des Chirurgen und eine unerwünschte Reduktion des Sehvermögens insbesondere hinsichtlich des Kontrastes bewirken, was möglichst effizient verhindert werden sollte.

Weiterhin zeigt die Fig. 1 zwei Halterungen 6,7, mittels derer die chirurgische Leuchte 1 im Bereich des Operationsfeldes an ein chirurgisches Instrument oder an einen Gegenstand im Operationsfeld lösbar befestigt werden kann. Zum einen ist eine Halterung 6 durch das zentral im Träger 5 beziehungsweise in der im Wesentlichen kreisförmigen Leiterplatte 5 angeordnete Loch 6 realisiert. Dieses Loch 6 zeigt einen darin liegenden gummielastischen Ring, der mit dem Träger 5 beziehungsweise der Leiterplatte 5 fest verbunden ist. Der freie Innendurchmesser des gummielastischen Rings ist dabei so gewählt, dass er wenig kleiner ist als der Außendurchmesser eines chirurgischen Schaftinstrumentes, wie zum Beispiel ein starres Endoskop oder eine minimalinvasive Zange oder ein Sauger oder Ähnliches, was einerseits zu einer guten Fixierung am Instrument durch das Einbringen des Schaftes des Instrumentes in das Loch 6 mit gummielastischem Ring als ergänzendes Halteelement führt und andererseits dennoch eine gewisse Beweglichkeit und damit Positionierbarkeit der chirurgischen Leuchte 1 am oder auf dem schaftförmigen Instrument in der Halterung 6 ermöglicht. Dies führt zu einer sehr guten Positionierbarkeit und damit Handhabbarkeit der chirurgischen Leuchte 1 im Bereich des Operationsfeldes.

Die weitere Halterung 7 dieser chirurgischen Leuchte 1 besteht aus einer Ausnehmung 7 im Randbereich der im Wesentlichen kreisförmigen Leiterplatte 5. Diese Ausnehmung 7 ist dabei mit zunehmendem Abstand vom Rand konisch verjüngend also keilförmig ausgebildet und geeignet, ein chirurgisches Instrument aufzunehmen. In dieser Position wird das Instrument dann mittels eines Bandes 7a, das mittels Klettverschluss an der Außenfläche der im Wesentlichen scheibenförmigen Leiterplatte 5 lösbar fixiert ist, positioniert und in dieser Position gehalten. Durch die Wahl der konisch verjüngenden Ausnehmung 7 erfolgt eine vorteilhafte Zentrierung der chirurgischen Leuchte 1 bezüglich des Instrumentes oder Objektes, an dem die chirurgische Leuchte 1 angebracht und dadurch gehalten wird, was für eine einfache und verlässliche Handhabung auch unter schwierigen Bedingungen wie im Operationsfeld besonders vorteilhaft ist. Die konische Ausnehmung 7 ermöglicht dabei eine formschlüssige Verbindung mit dem Instrument, wobei durch das Zusammenwirken mit dem Band 7a eine sichere Positionierung erreicht wird. Durch die Zentrierung reicht eine grobe Grundpositionierung der chirurgischen Leuchte 1 mit der anschließenden Zentrierung beim weiteren Einbringen in die Ausnehmung für eine sicherere und präzise Positionierung mit der anschließenden Fixierung durch das Band 7a aus.

Durch das Vorsehen beider Halterungen 6,7 ist es möglich, eine besonders sichere Positionierung und Fixierung der chirurgischen Leuchte 1 an einem oder mehreren Instrumenten oder Objekten im Operationsfeld zu schaffen.

Auf der Vorderseite 5a der Leiterplatte 5 sind mehrere Leiterbahnen 4 zur elektrischen Energieversorgung der als ungehäuste LEDs 2 ausgebildeten Lichtquellen 2 angeordnet. Die Leiterbahnen 4 werden dabei von einer oder mehreren elektrischen Stromversorgungen 3 auf der Rückseite 5b der Leiterplatte 5 mit elektrischer Energie versorgt.

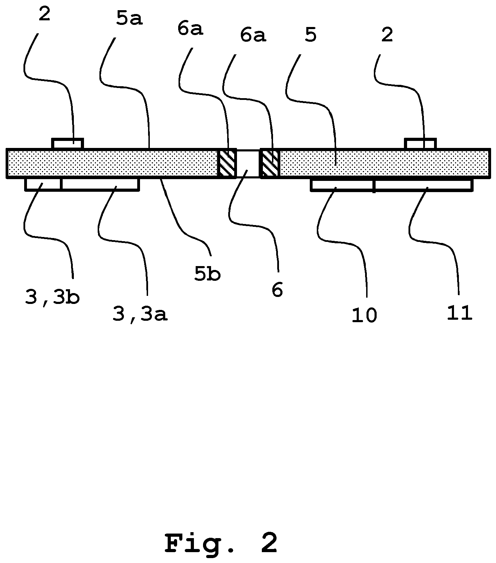

Dies ist in Fig. 2 dargestellt. In der schematischen Darstellung eines Schnittes durch die Leiterplatte 5 entlang der Linie A - A in Fig. 1 sind die als ungehäuste LEDs 2 ausgebildeten Lichtquellen 2 auf der Vorderseite 5a der Leiterplatte 5 zu erkennen. Diesen gegenüber auf der Rückseite 5b der Leiterplatte 5 ist zum einen die Stromversorgung 3 angeordnet. Diese Stromversorgung 3 zeigt eine Steckeraufnahme 3a, an die sich radial nach außen ein elektrischer Energiespeicher, ein sogenannter Goldcap 3b, anschließt. Auf der dem Operateur zugewandten Rückseite 5b und damit auf der dem zu beleuchtenden Operationsfeld abgewandten Seite der chirurgischen Leuchte 1 kann bei Bedarf die dem Chirurgen zugängliche Steckeraufnahme 3a dazu verwendet werden, dass ein elektrisches Energieversorgungskabel angeschlossen wird, um die chirurgische Leuchte 1 mit elektrischer Energie für die Lichtquellen 2 zu versorgen. Durch das Vorsehen des als Goldcap ausgebildeten elektrischen Energiespeichers 3b ist es ermöglicht, die über ein elektrisches Kabel zugeführte Energie zu puffern, so dass die chirurgische Leuchte 1 auch ohne angeschlossenes elektrisches Kabel für eine relevante Zeit autonom funktionsfähig ist und für eine ausreichende Operationszeit das Operationsfeld wunschgemäß beleuchten kann.

Durch die Positionierung der Lichtquellen 2 auf der Vorderseite 5a und der Stromversorgung 3 mit der Steckeraufnahme 3a auf der Rückseite 5b ist eine besonders gute Handhabung des Chirurgen oder seiner Hilfskräfte auch unter den schwierigen Bedingungen im Bereich des Operationsfeldes besonders verlässlich möglich, was die Sicherheit der chirurgischen Leuchte 1 besonders stärkt.

In entsprechender Weise sind auf der Rückseite 5b die Steuerung 10, die in Form eines integrierten Schaltkreises realisiert ist, und eine Antenne 11, die den drahtlosen Empfang von Steuersignalen von einer abgesetzten Bedieneinheit ermöglicht, angeordnet. Die empfangenen Steuersignale werden von der Antenne 11, die vorzugsweise als Kopplungselement für eine induktive Kopplung ausgebildet ist, an die Steuerung 10 weitergeleitet, die die empfangenen Signale bei Bedarf verstärkt und in interne Steuersignale zur Steuerung 10 der chirurgischen Leuchte 1 so umsetzt, dass die Lichtquellen 2 zielgerichtet ein- und ausgeschaltet beziehungsweise in ihrer Helligkeit verändert beziehungsweise in ihrem Farbton und damit in der Farbverteilung beziehungsweise Frequenzverteilung oder Wellenlängenverteilung des Lichtes angepasst werden können. Durch die Anordnung der Antenne 11 auf der Rückseite 5b ist ein besonders guter Empfang der drahtlos übertragenen Steuersignale von der abgesetzten Bedieneinheit im Umfeld des Chirurgen geschaffen, ohne dass der Empfang beispielsweise durch die Leiterplatte 5 abgeschirmt oder behindert wird. Durch die bevorzugte Trennung der Lichtquellen 2 auf der Vorderseite 5a von den anderen Komponenten auf der Rückseite 5b gelingt es, eine sehr vorteilhafte Verteilung der Lichtquellen 2 im Hinblick auf eine optimierte Beleuchtung zu ermöglichen, ohne dass wichtige Positionen auf der Vorderseite 5a durch die anderen Komponenten wie zum Beispiel die Stromversorgung 3 oder die Steuerung 10 oder die Antenne 11 oder durch Bedienelemente auf dem Träger 5 beziehungsweise der Leiterplatte 5 blockiert werden.

In der Mitte des in Fig. 2 dargestellten Schnittes durch die Leiterplatte 5 ist das durchgängige Loch 6 angeordnet, das durch den gummielastischen Ring 6a als Teil des gemeinsamen Halteelements 6 begrenzt ist und im Durchmesser und in seiner Länge so gewählt ist, dass ein Schaft eines chirurgischen Instrumentes verlässlich gehalten wird, ohne dass eine Feinpositionierung oder Repositionierung der chirurgischen Leuchte 1 verhindert wird. Dabei hat es sich als besonders vorteilhaft erwiesen, dass der gummielastische Ring 6a in seiner Länge wenigstens gleich der Dicke der Leiterplatte 5 oder etwa gleich einem Mehrfachen der Dicke der Leiterplatte 5 gewählt wird, was zu einer guten Fixierung bei der gewünschten Repositioniermöglichkeit führt. Dabei ist der Innendurchmesser des gummielastischen Rings 6a wenig geringer als der Außendurchmesser des Schafts des chirurgischen Instrumentes gewählt.

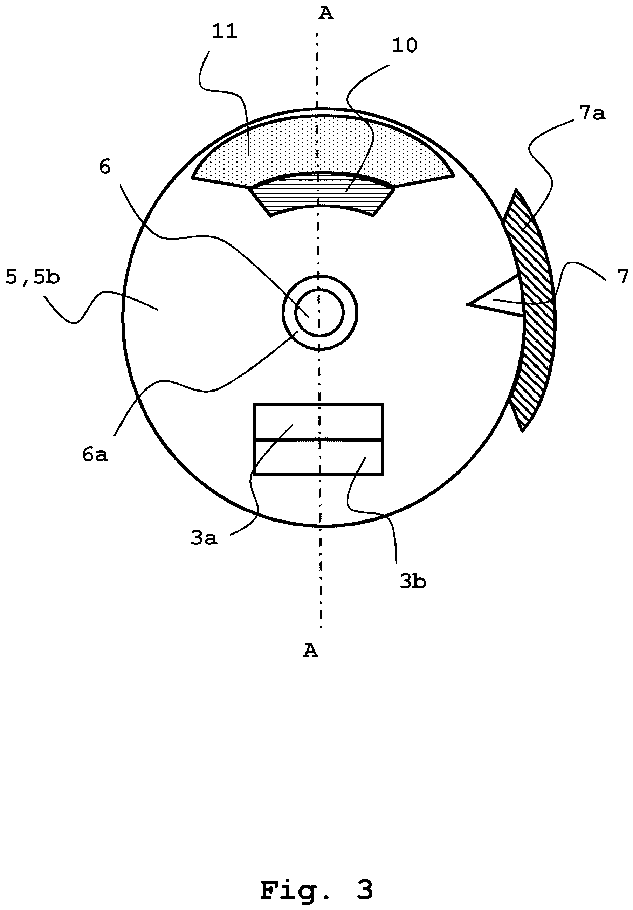

Fig. 3 zeigt in einer Draufsicht die Rückseite 5b der Leiterplatte 5 der chirurgischen Leuchte 1. Sie zeigt die Halterungen 6,7 zur Aufnahme chirurgischer Instrumente entsprechend der in Fig. 11 dargestellten Vorderseite 5a der Leiterplatte 5 der chirurgischen Leuchte 1. Dabei sind die Steuerung 10 und die Antenne 11 flächig als integrierte Schaltkreise auf der Leiterplatte 5 aneinanderstoßend und elektrisch wie signaltechnisch verbunden angeordnet. Gegenüber der Steuerung 10 und der Antenne 11 ebenso auf der Rückseite 5b sind die Steckeraufnahme 3a und direkt benachbart der elektrische Energiespeicher 3b in Form eines Goldcaps 3b angeordnet. Diese Komponenten nehmen nur eine geringe Fläche der Rückseite 5b ein, so dass die Möglichkeit besteht, auf der Rückseite 5b zusätzliche Bedienelemente insbesondere in Form von Annäherungsschaltern anzuordnen, die ein Ansteuern und Bedienen der chirurgischen Leuchte 1 mit ihren Lichtquellen 2 auf der Vorderseite 5a ermöglichen.

Diese chirurgische Leuchte 1 zeichnet sich durch eine besonders vorteilhafte Lichtverteilung mit ausreichender Lichtstärke, mit gleichmäßiger insbesondere schattenarmer Lichtverteilung, mit einem hohen Maß an Energieeffizienz und geringer Wärmeentwicklung aus. Sie zeigt sich darüber hinaus im Hinblick auf die Handhabung als sehr gut zu handhaben und als sehr verlässlich, was gerade dann der Fall ist, wenn die chirurgische Leuchte 1 ohne zusätzliche Kabel betrieben wird. Auch erweist sie sich gerade für den Einsatz im Operationsfeld als besonders geeignet, da sie sich durch die verwendeten Materialien und die Anordnungen insbesondere mit den Kapselungen und den wenigen Komponenten als sehr gut sterilisierbar erweist und dadurch das Risiko von Kontaminierungen besonders niedrig gehalten wird.

### Bezugszeichenliste

- 1: Chirurgische Leuchte
- 2: Lichtquelle
- 3: Elektrische Stromversorgung
- 3a: Steckeraufnahme
- 3b: elektrischer Energiespeicher, Goldcap
- 4: Leiterbahn
- 5: Träger, Leiterplatte
- 5a: Vorderseite
- 5b: Rückseite
- 6: Halterung, zentrales Loch
- 6a: elastischer Ring
- 7: Halterung, Ausnehmung
- 7a: bandförmiger Verschluss
- 10: Steuerung
- 11: Antenne, Koppungselement für induktive Kopplung

## Patentansprüche

1. Chirurgische Leuchte (1), die mobil ausgebildet ist, die wenigstens eine Lichtquelle (2) zur Beleuchtung des Operationsfeldes aufweist,
die mit einer elektrischen Stromversorgung (3) für die wenigstens eine Lichtquelle (2) verbunden ist,
wobei die Chirurgische Leuchte (1) zur lösbaren Befestigung an einem chirurgischen Instrument oder einem Gegenstand im Operationsfeld geeignet ist, wobei die Chirurgische Leuchte (1) einen Träger (5) zur Aufnahme der wenigstens einen Lichtquelle (2) aufweist, der als Leiterplatte (5) mit einer Vorderseite (5a) und einer zweiten Seite (5b) ausgebildet ist,
wobei auf der Vorderseite (5a) die wenigstens eine Lichtquelle (2) angeordnet ist
und wobei der Träger (5) mit einer Halterung (6,7) zur lösbaren Verbindung mit einem chirurgischen Instrument oder einem Gegenstand im Operationsfeld versehen ist
**dadurch gekennzeichnet,**
**dass** der Träger (5) als Leiterplatte mit im Wesentlichen rundem insbesondere ovalem oder kreisrundem Querschnitt ausgebildet ist.

2. Chirurgische Leuchte (1) nach Anspruch 1, wobei die Halterung (6,7) wenigstens ein Loch (6) im Träger(5), wenigstens einen Clip, wenigstens einen Magneten, wenigstens ein Formschlusselement (7), wenigstens ein elastisches Halteelement, wenigstens eine Klebefläche und/oder wenigstens ein Klettverschlusselement (7a) aufweist.

3. Chirurgische Leuchte (1) nach einem der Ansprüche 1 oder 2, wobei die Halterung (6,7) neben wenigstens einem Loch (6) im Träger(5) wenigstens einen Clip, wenigstens einen Magneten, wenigstens ein Formschlusselement (7), wenigstens ein elastisches Halteelement, wenigstens eine Klebefläche und/oder wenigstens ein Klettverschlusselement (7a) aufweist.

4. Chirurgische Leuchte (1) nach einem der Ansprüche 1 bis 3, wobei der Träger (5) als Leiterplatte mit im Wesentlichen ovalem oder kreisrundem Querschnitt ausgebildet ist.

5. Chirurgische Leuchte (1) nach einem der Ansprüche 1 bis 4, wobei auf der zweiten Seite insbesondere auf der Rückseite (5b) des Trägers (5) die Stromversorgung (3) und/oder eine Steuerung (10) der Leuchte (1) angeordnet sind.

6. Chirurgische Leuchte (1) nach einem der Ansprüche 1 bis 5, wobei der Träger (5) mit wenigstens einer chemisch inerten oder flüssigkeitsdichten oder sterilisierbaren Kapselung für die wenigstens eine Lichtquelle (2), die Stromversorgung (3), die Steuerung (10) und/oder Teile davon insbesondere mittels einer Vergussmasse versehen ist.

7. Chirurgische Leuchte (1) nach Anspruch 6, wobei auf der zweiten Seite insbesondere auf der Rückseite (5b) des Trägers (5) wenigstens eine Zusatzlichtquelle angeordnet ist, wobei diese Zusatzlichtquelle insbesondere eine deutlich geringere Lichtstärke als die Lichtquelle (2) aufweist.

8. Chirurgische Leuchte (1) nach einem der Ansprüche 1 bis 7, wobei die Leiterplatte (5) mit einer Dicke von wenigen Millimetern und aus Metall und/oder Kunststoff und/oder Keramik gebildet ist .

9. Chirurgische Leuchte (1) nach einem der Ansprüche 1 bis 8, wobei die Stromversorgung (3) unter Verwendung eines elektrischen Kabels und/oder einer drahtlosen, induktiv gekoppelten Verbindung und/oder eines elektrischen Energiespeichers (3b) insbesondere eines Kondensators oder eines Akkumulators gebildet ist.

10. Chirurgische Leuchte (1) nach einem der Ansprüche 1 bis 9, wobei die wenigstens eine Lichtquelle (2) zur Beleuchtung des Operationsfeldes wenigstens eine Laserdiode, wenigstens eine OLED und/oder wenigstens eine LED insbesondere wenigstens einen ungehäusten LED-Chip aufweist.

11. Chirurgische Leuchte (1) nach Anspruch 10, wobei mehrere Laserdioden, OLEDs und/oder LEDs insbesondere mehrere ungehäuste LED-Chips auf der Oberfläche insbesondere auf der Vorderseite (5a) des Trägers (5) voneinander beabstandet verteilt angeordnet sind und insbesondere mit einer gemeinsamen und chemisch inerten und/oder flüssigkeitsdichten und/oder sterilisierbaren Kapselung versehen sind.

12. Chirurgische Leuchte (1) nach einem der Ansprüche 1 bis 11, wobei wenigstens eine Kapselung für die wenigstens eine Lichtquelle (2) vorgesehen ist und wobei wenigstens eine Kapselung als optisches Element zur Lenkung insbesondere zur Bündelung der von der wenigstens einen gekapselten Lichtquelle (2) ausgesandten Strahlung ausgebildet ist.

13. Chirurgische Leuchte (1) nach einem der Ansprüche 1 bis 12, wobei eine Steuerung (10) der Leuchte (1) vorgesehen ist, die ein Aus- und Einschalten, ein Dimmen und/oder eine Änderung der Farbe des ausgesandten Lichtes der Leuchte (1) ermöglicht.

14. Chirurgische Leuchte (1) nach einem der Ansprüche 1 bis 13, wobei eine Bedieneinheit der chirurgischen Leuchte (1) vorgesehen ist, die auf dem Träger(5), in einer elektrischen Leitung zur Versorgung der Leuchte (1) mit elektrischer Energie und/oder bei einer abgesetzten elektrischen Stromversorgung (3) für die restliche Leuchte (1) angeordnet ist.

15. Chirurgische Leuchte (1) nach einem der Ansprüche 1 bis 14, wobei die Bedieneinheit der chirurgischen Leuchte (1) mit wenigstens einem Bedienschalter insbesondere einem mechanischen Bedienschalter, einem Berührschalter und/oder Annäherungsschalter versehen ist und/oder die Bedieneinheit (3) abgesetzt und kabellos mit der Steuerung (10) zum Ansteuern der wenigstens einen Lichtquelle (2) der chirurgischen Leuchte (1) verbunden ist.
